# EUROPEAN PATENT APPLICATION

(11) **EP 4 582 532 A1**
(43) Date of publication of application: **09.07.2025**
(21) Application number: 23860350.0
(22) Date of filing: 29.08.2023
(51) Int. Cl.: C12M 3/00, C12N 5/071, A23L 13/00

(54) **CELL CULTURE SYSTEM, CULTURED MEAT, AND CULTURE SUPERNATANT**

(30) Priority: 31.08.2022 JP 2022137890
(71) Applicant: Integriculture Inc., Tokyo 113-0033 (JP)
(72) Inventor: OCHIAI Toshiro, Tokyo 113-0033 (JP); KOKIDO Ibuki, Tokyo 113-0033 (JP)
(74) Representative: V.O.
(86) International application number: PCT/JP2023/031244
(87) International publication number: WO 2024/048588

(57) **Abstract**

A new cell culture system capable of improving the efficiency in cell culture is provided. A cell culture system (S1) comprises a plurality of cell culture devices (100, 200, 300) which are connected such that a culture medium (50) moves through each of the cell culture devices, and the liquid level of the culture medium stored in each of the cell culture devices is controlled such that cells in at least one of the cell culture devices are in a first state in which the cells are immersed in the culture medium, while cells in the remaining cell culture devices are in a second state in which the cells are not immersed in the culture medium.

## Description

### TECHNICAL FIELD

The present invention relates to a cell culture system configured with a plurality of cell culture devices which are connected with each other, and cultured meat and culture supernatant produced by the cell culture system.

### BACKGROUND ART

In recent years, the development of a truly sustainable food production system is spreading worldwide to achieve the "Sustainable Development Goals (SDGs)" and, in particular, the development relating to "cultured meat" has been active. As a background art of this technical field, for example, Patent Literature 1 discloses a cell culture device including a first chamber and a second chamber. In Patent Literature 1, the second chamber is designed, for example, in the form of a bellow, and compressing and decompressing the second chamber using a volume-adjusting means allows the liquid and the air to flow between the chambers.

Furthermore, in the cell culture device according to Patent Literature 1, a growth substrate means for cell attachment and growth is provided in the first chamber. The growth substrate means is intermittently and periodically, but indirectly, exposed to a gaseous environment via a thin gas-growth medium interface in order to receive oxygen or be submerged in culture medium in accordance with the compression and decompression of the second chamber. This facilitates cell growth and cellular product production.

### CITATION LIST

### PATENT LITERATURE

Patent Literature 1: JP-B-4430317

### SUMMARY OF INVENTION

### TECHNICAL PROBLEM

However, Patent Literature 1 merely discloses the configuration of a cell culture device itself and a method of using (controlling) the cell culture device, however, does not disclose any specific configuration of a cell culture system configured with a combination of a plurality of cell culture devices and a method of controlling the same.

The present invention has been made in view of such circumstances described above, and the main object thereof is to provide a new cell culture system capable of improving the efficiency in cell culture. A further object of the present invention is to provide culture meat and culture supernatant produced by the cell culture system.

### SOLUTION TO PROBLEM

In order to achieve the objects described above, a first aspect of the present invention is a cell culture system comprising a plurality of cell culture devices which are connected such that a culture medium moves through each of the plurality of cell culture devices, wherein the liquid level of the culture medium stored in each of the plurality of cell culture devices is controlled such that cells in at least one of the plurality of cell culture devices are in a first state, while cells in the remaining cell culture devices are in a second state, the first state being a state in which the cells are immersed in the culture medium, and the second state being a state in which the cells are not immersed in the culture medium.

A second aspect of the present invention is cultured meat produced by the cell culture system.

A third aspect of the present invention is culture supernatant produced by the cell culture system.

### ADVANTAGEOUS EFFECTS OF INVENTION

According to the present invention, it is possible to provide a new cell culture system capable of improving the efficiency in cell culture. Furthermore, it is possible to provide cultured meat and culture supernatant produced by the cell culture system. The problems, configurations, and advantageous effects other than those described above will be clarified by explanation of the embodiments below.

### BRIEF DESCRIPTION OF DRAWINGS

[FIG. 1] FIG. 1 illustrates an overall configuration of a cell culture system according to the first embodiment.
[FIG. 2] FIG. 2 schematically illustrates a longitudinal section of a cell culture device.
[FIG. 3] FIG. 3 schematically illustrates a cell culture device as viewed from above.
[FIG. 4] FIG. 4 illustrates the details of a shelf as viewed from above.
[FIG. 5] FIG. 5 illustrates results of a cell culture experiment.
[FIG. 6] FIG. 6 illustrates results of an experiment on the number of cells and glucose consumption.
[FIG. 7] FIG. 7 illustrates an overall configuration of a cell culture system according to the second embodiment.

### DESCRIPTION OF EMBODIMENTS

Hereinafter, with reference to the drawings, the embodiments of the present invention will be described using the case of cultivating animal cells.

### (First embodiment)

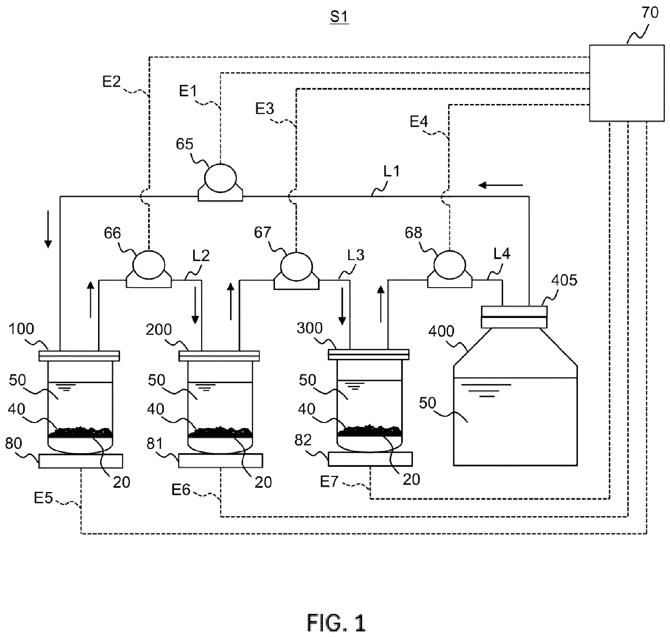

A cell culture system S1 according to the first embodiment of the present invention will be described. FIG. 1 illustrates an overall configuration of the cell culture system S1 according to the first embodiment. The cell culture system S1 illustrated in FIG. 1 is configured with, for example, three cell culture devices 100, 200, 300 and a buffer tank 400, which are connected in a loop (in series) via flow paths L1 to L4. Here, the flow paths L1 to L4 are, for example, tubes or hoses.

The cell culture devices 100, 200, 300 are placed on balances 80, 81, 82, respectively. The cell culture devices 100, 200, 300 are supplied with different animal cells, respectively. Animal cells are, for example, muscle-derived cells, skin-derived cells, intestine-derived cells, heart-derived cells, brain-derived cells, stomach-derived cells, embryonic membrane-derived cells, liver-derived cells, kidney-derived cells, and lung-derived cells. In the present embodiment, the cell culture devices 100, 200, 300 are supplied with, for example, lung-derived cells, liver-derived cells, and muscle-derived cells, respectively.

The buffer tank 400 is a container made of stainless steel or glass for storing culture supernatant. Closing the lid 405 causes the inside of the buffer tank 400 to be sealed.

The flow paths L1 to L4 are provided with pumps 65 to 68, respectively. The pumps 65 to 68 are driven by motors (not illustrated), respectively. The flow rate of the pumps 65 to 68 changes in accordance with the change in the rotational speed of the motor.

The pump 65 sucks culture medium 50 in the buffer tank 400 and discharges it toward the cell culture device 100. The pump 66 sucks the culture medium 50 in the cell culture device 100 and discharges it toward the cell culture device 200. The pump 67 sucks the culture medium 50 in the cell culture device 200 and discharges it toward the cell culture device 300. The pump 68 sucks the culture medium 50 in the cell culture device 300 and discharges it toward the buffer tank 400. With this configuration, driving all the pumps 65 to 68 causes the culture medium 50 to circulate in the direction illustrated with the arrow in FIG. 1.

A controller 70 is connected to the pumps 65 to 68 (more specifically, motors for driving the pumps 65 to 68) via electric wires E1 to E4, and also connected to the balances 80, 81, 82 via electric wires E5 to E7. The controller 70 receives weight data about the cell culture devices 100, 200, 300 detected by the balances 80, 81, 82 via the electric wires E5 to E7. Furthermore, the controller 70 is electrically connected to various sensors (not illustrated) mounted to the cell culture devices 100, 200, 300, respectively, and receives sensor data (for example, the temperature and pH value of the culture medium) detected by the various sensors.

The controller 70 is configured with a processor including a CPU, a ROM and a RAM which serve as storage devices, and peripheral circuits. A control program is stored in the ROM, and the CPU reads the control program to execute various processes. The controller 70 controls, for example, driving of the pumps 65 to 68 based on the various types of information as received (details thereof will be described later).

Next, the structures of the cell culture devices 100, 200, 300 will be described in detail. The structure of the cell culture device 100 and those of the cell culture devices 200, 300 are the same except that the types of animal cells to be cultured therein are different from each other and thus, explanation for the structures of the cell culture devices 200, 300 will be omitted herein.

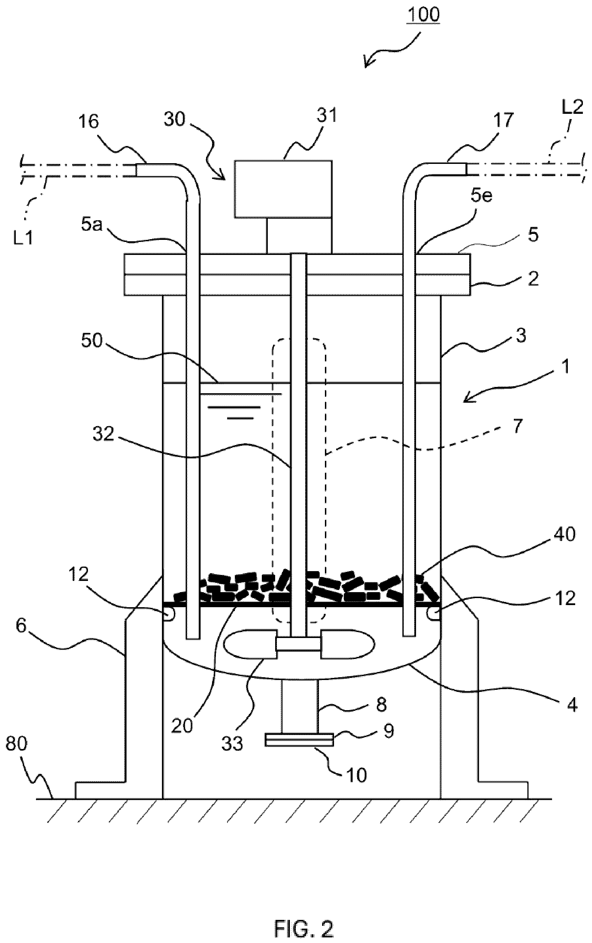

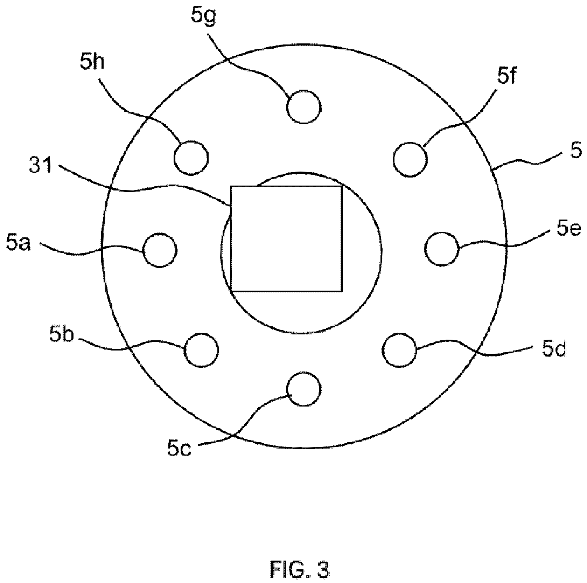

FIG. 2 schematically illustrates the longitudinal section of the cell culture device 100, and FIG. 3 schematically illustrates the cell culture device 100 as viewed from above. As illustrated in FIG. 2 and FIG. 3, the cell culture device 100 includes a culture tank 1, a shelf 20, and a stirring means 30.

The culture tank 1 includes a bottomed cylindrical container formed with a body portion 3 and a bottom portion 4, and a disk-shaped lid body 5 for covering the top of the body portion 3. The culture tank 1 stores the culture medium 50 therein. The culture tank 1 is made of, for example, stainless steel. The body portion 3 is provided on its top with a flange 2 extending outward in the radial direction, and the disk-shaped lid body 5 is mounted to the flange 2 via a seal member such as an O-ring (not illustrated). With this structure, the inside of the culture tank 1 is sealed. In the present embodiment, wing bolts are used for fixing between the flange 2 and the lid body 5, however, other fixing means may be used instead thereof.

The culture tank 1 is provided on its bottom portion 4 with a waste liquid nozzle 8 serving as a drain. The waste liquid nozzle 8 is provided on its lower end with a flange 9 extending outward in the radial direction, to which a blind flange 10 is mounted via a seal member such as an O-ring. In a normal state, the blind flange 10 closes the waste liquid nozzle 8. Then, removing the blind flange 10 causes the culture medium 50 stored in the culture tank 1 to be discharged therefrom. Of course, a valve such as a gate valve may be used in place of the blind flange 10, in this case, opening and closing the valve causes the culture medium 50 to be discharged.

The body portion 3 of the culture tank 1 is provided with an elongated viewing window 7 extending in the vertical direction. The viewing window 7 is made of, for example, a transparent glass plate, and the inside of the culture tank 1 can be viewed through the viewing window 7. For example, a user can check the liquid level of the culture medium 50 or observe the state of a scaffold 40 (growth state of animal cells), which will be described later, through the viewing window 7.

Furthermore, the body portion 3 is provided at its lower part of the inner curved surface with a plurality of (for example, four) protrusions (holding portions) 12, and the protrusions 12 are arranged at the same height positions along the circumferential direction. Each of the protrusions 12 is formed to be large enough to support the shelf 20, which will be described later, from below. That is, the protrusions 12 function as stoppers for limiting the position of the shelf 20 in the vertical direction. As long as the shelf 20 can be held at a predetermined height position, it may include, for example, legs instead of the protrusions 12, or be suspended from the lid body 5.

Furthermore, the body portion 3 is provided at its lower part of the outer curved surface with a plurality of (for example, four) legs 6. The culture tank 1 is supported by these legs 6. In the case of the culture tank 1 having a large size, a skirt may be used instead of the legs 6.

In the following, the lid body 5 will be described. As illustrated in FIG. 3, the lid body 5 is provided with a plurality of (for example, eight) mounting holes 5a to 5h along the circumferential direction. The mounting holes 5a to 5h are provided with various sensors, pipes, and the like, respectively. As illustrated in FIG. 2, for example, the mounting hole 5a is provided with a culture medium supply pipe 16 for supplying the culture medium 50 to the culture tank 1, and the mounting hole 5e is provided with a culture medium transfer pipe 17 for transferring the culture medium 50 stored in the culture tank 1 to the outside (specifically, to the cell culture device 200). The culture medium supply pipe 16 is connected to the flow path L1, and the culture medium transfer pipe 17 is connected to the flow path L2. With this configuration, as illustrated in FIG. 1, the culture medium 50 discharged by the pump 65 flows through the flow path L1, and is supplied from the culture medium supply pipe 16 to the culture tank 1 (cell culture device 100). Upon being sucked by the pump 66, the culture medium 50 in the culture tank 1 flows through the flow path L2 via the culture medium transfer pipe 17, and is transferred to the cell culture device 200.

Here, as illustrated in FIG. 2, the lower end portion of the culture medium supply pipe 16 extends to a position below the shelf 20. This allows the culture medium 50 to be stored in the culture tank 1 without shaking the liquid surface of the culture medium 50 so much when the culture medium 50 is supplied to the culture tank 1. Furthermore, the lower end portion of the culture medium transfer tube 17 also extends to a position below the shelf 20. This allows the culture medium 50 to be transferred to the outside until the liquid level of the culture medium 50 reaches a position below the shelf 20. When the liquid level of the culture medium 50 lowers below the shelf 20, the shelf 20 (scaffold 40 placed on the shelf 20) is no longer immersed in the culture medium 50 and thus exposed to air.

The mounting holes 5a to 5h are provided with, in addition to the culture medium supply pipe 16 and the culture medium transfer pipe 17, any sensor or pipe necessary for culturing animal cells, or any plugs for ventilation. Such sensors and pipes are, for example, sensors for detecting a desired physical quantity such as the component, temperature, pH, and content of protein of the culture medium 50, pipes such as a sampling pipe, animal cell supply pipe, alkaline supply pipe, and oxygen supply pipe, and the like. Furthermore, the lid body 5 is provided with a motor 31 of the stirring means 30, which will be described later, and the motor 31 is fixed to a central portion of the lid body 5. Although not illustrated, the cell culture device 100 is provided with a pipe that communicates with the outside air, and this pipe is provided with a filter to prevent the entry of microorganisms or minute objects from the outside air.

Next, the shelf 20 will be described. The shelf 20 is a disk-shaped member on which the scaffold 40, which will be described later, is to be placed. The shelf 20 is supported by the four protrusions 12 so as to be held at a predetermined height position from the bottom portion 4 of the culture tank 1, and partitions the space in the culture tank 1 into two in the height direction (vertical direction). The predetermined height position can be appropriately set in consideration of the volume of the culture tank 1, the amount of the culture medium 50, and the like, however, for practical use, it is preferable to set the height position to approximately 1/3 of the length of the body portion 3 from the bottom portion 4 of the culture tank 1.

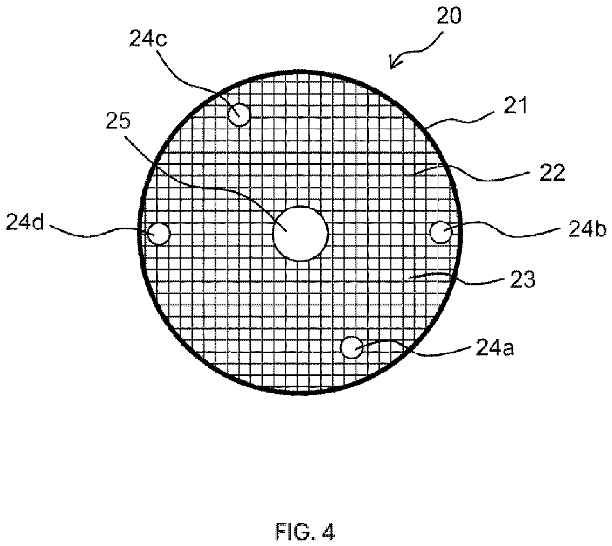

FIG. 4 illustrates the details of the shelf 20 as viewed from above. As illustrated in FIG. 4, the shelf 20 includes a ring 21 serving as an outer frame, and a wire mesh 22 formed by weaving, for example, a wire member made of stainless steel to form a grid pattern. The wire mesh 22 includes a plurality of nets (openings) 23, each of which has the size allowing the culture medium 50 to pass therethrough. The mesh size (that is, the size of each net) of the wire mesh 22 is appropriately set based on the shape of the scaffold 40, the size of animal cells to be attached to the scaffold 40, the viscosity and specific gravity of the culture medium 50, and the like. In the present embodiment, for example, the wire mesh 22 having 100 to 150 meshes (the number of nets per inch is 100 to 150) is used.

Furthermore, the wire mesh 22 includes a central hole 25 through which a rotation shaft 32 of the stirring means 30, which will be described later, can be inserted, and small holes 24a to 24d through which, the culture medium supply pipe 16, the culture medium transfer pipe 17, and the like can be inserted.

On the shelf 20, the scaffold 40 is to be placed. The scaffold 40 allows animal cells to be attached thereto, and has a three-dimensional porous structure. In the present embodiment, the scaffold 40 is edible. The scaffold 40 includes, for example, proteins and polysaccharides. Here, the term "edible" refers to being a composition that can be safely ingested into the body. Animal cells adhere to the scaffold 40, whereby cultured meat is produced.

Next, the stirring means 30 will be described. The stirring means 30 is provided to stir the culture medium 50, and, as illustrated in FIG. 2, includes the motor 31 as a driving source, the rotation shaft 32, and stirring blades (stirring portion) 33. That is, in the present embodiment, the stirring means 30 with a propeller is used.

The motor 31 is fixed to the lid body 5. The output shaft of the motor 31 is connected to the rotation shaft 32 via a coupling (not illustrated). The rotation shaft 32 is provided on its distal end with the plurality of (for example, three) stirring blades 33. In response to the rotation of the motor 31, the rotation shaft 32 rotates and thus the three stirring blades 33 rotate in the horizontal direction about the rotation shaft 32. This causes the culture medium 50 to be stirred.

Here, the stirring blades 33 are placed in the space between the shelf 20 and the bottom portion 4. In this arrangement, rotation of the stirring blades 33 causes the culture medium 50 to be gradually stirred from the lower side of the culture tank 1. In the present embodiment, the motor 31 is capable of rotating in both directions, and thus rotates in the forward or reverse direction at a predetermined cycle and a predetermined rotational speed based on a control signal from the controller 70 (see FIG. 1). However, a motor 31 that rotates in one direction may be used.

Next, operation modes provided in the cell culture system S1 will be described.

The cell culture system S1 includes a first operation mode for stopping the pumps 65 to 68 sequentially one by one and driving the remaining pumps simultaneously at a predetermined flow rate, and second to fourth operation modes for temporarily stopping only a particular one of the pumps 65 to 68. The controller 70 controls the cell culture system S1 in accordance with the first to fourth operation modes. In the following, the first to fourth operation modes will be described in detail.

### (First operation mode)

The first operation mode is an operation mode for circulating the culture medium 50 while temporarily stopping the driving of the pump 65, the pump 66, the pump 67, and the pump 68 repeatedly in this order. When the controller 70 temporarily stops the pump 65, the supply of the culture medium 50 from the buffer tank 400 to the cell culture device 100 is stopped. During this time, the culture medium 50 in the cell culture device 100 is sucked by the pump 66 as the controller 70 drives the remaining pumps 66, 67, 68, which lowers the liquid level of the culture medium 50 of the cell culture device 100. Then, when the liquid level of the culture medium 50 of the cell culture device 100 reaches a predetermined height position which is below the shelf 20, the scaffold 40 placed on the shelf 20 is no longer immersed in the culture medium 50 and thus exposed to the air (second state). At this time, the liquid level of the culture medium 50 of each of the other cell culture devices 200, 300 is positioned above the shelf 20 (first state).

Next, the controller 70 temporarily stops the pump 66 and drives the remaining pumps 65, 67, 68, whereby the supply of the culture medium 50 to the cell culture device 100 starts again. On the other hand, the stop of the supply of the culture medium 50 to the cell culture device 200 causes the liquid level of the culture medium 50 of the cell culture device 200 to be gradually lowered and thus, the scaffold 40 placed on the shelf 20 is exposed to the air. As described above, the controller 70 repeatedly pauses the pumps 65, 66, 67, 68 in order so as to sequentially lower the liquid level of the culture medium 50 of each of the cell culture devices 100, 200, 300. That is, the first operation mode is a mode for bringing the scaffolds 40 in the cell culture devices 100, 200, 300 to be in the states of being exposed to the air sequentially by temporarily stopping the pumps 65, 66, 67, 68 in order. In the present embodiment, the period of time during which the scaffold 40 is exposed to the air is set to approximately 30 seconds to 180 seconds.

Here, the controller 70 determines that the liquid level of the culture medium 50 reaches a predetermined position which is below the shelf 20 based on the weight data from the balances 80, 81, 82. Specifically, the weight of each of the cell culture devices 100, 200, 300 when the liquid level of the culture medium 50 reaches the predetermined position is stored in the controller 70 as a predetermined threshold value, and the controller 70 determines that the liquid level of the culture medium 50 of the cell culture devices 100, 200, 300 has reached a position which is below the shelf 20 when the weight data as received becomes equal to or less than the threshold value. In the present embodiment, the height position of the liquid level of the culture medium 50 is detected using the balances 80, 81, 82, however, it may be detected, instead thereof, using a level sensor, an ultrasonic liquid level sensor, or the like.

### (Second operation mode)

The second operation mode is a mode for lowering only the liquid level of the culture medium 50 in the cell culture device 100 so that the scaffold 40 thereof is temporarily exposed to the air, and causing the scaffolds 40 of the other cell culture devices 200, 300 to be constantly immersed in the culture medium 50.

Specifically, the controller 70 temporarily stops the pump 65 while driving the pumps 66, 67, 68. Then, when the liquid level of the culture medium 50 of the cell culture device 100 reaches a height position which is below the shelf 20, the controller 70 stops all the pumps 65 to 68 for a predetermined period of time (for example, 1 minute) so that only the scaffold 40 of the cell culture device 100 is exposed to the air. At this time, in the cell culture devices 200, 300, the scaffolds 40 are immersed in the culture medium 50, respectively. After the predetermined period of time passes, the controller 70 executes the control of, firstly, driving the pump 65 to cause the cell culture device 100 to be filled with the culture medium 50, and then driving the pumps 66, 67, 68 to cause the culture medium 50 to circulate through each of the cell culture devices 100, 200, 300. As described above, the second operation mode is an operation mode for causing only the scaffold 40 in the cell culture device 100 to be temporarily exposed to the air, while causing the scaffolds 40 of the other cell culture devices 200, 300 to be constantly immersed in the culture medium 50.

### (Third operation mode)

The third operation mode is a mode for lowering only the liquid level of the culture medium 50 in the cell culture device 200 so that the scaffold 40 thereof is temporarily exposed to the air, and causing the scaffolds 40 of the other cell culture devices 100, 300 to be constantly immersed in the culture medium 50.

Specifically, the controller 70 temporarily stops the pumps 65, 66 while driving the pumps 67, 68. Then, when the liquid level of the culture medium 50 of the cell culture device 200 reaches a height position which is below the shelf 20, the controller 70 stops all the pumps 65 to 68 for a predetermined period of time (for example, 1 minute) so that only the scaffold 40 of the cell culture device 200 is exposed to the air. At this time, in the cell culture devices 100, 300, the scaffolds 40 are immersed in the culture medium 50, respectively. After the predetermined period of time passes, the controller 70 executes the control of, firstly, driving the pumps 65, 66 to cause the cell culture device 200 to be filled with the culture medium 50, and then driving the pumps 67, 68 to cause the culture medium 50 to circulate through each of the cell culture devices 100, 200, 300. As described above, the third operation mode is an operation mode for causing only the scaffold 40 in the cell culture device 200 to be temporarily exposed to the air while causing the scaffolds 40 of the other cell culture devices 100, 300 to be constantly immersed in the culture medium 50.

### (Fourth operation mode)

The fourth operation mode is a mode for lowering only the liquid level of the culture medium 50 in the cell culture device 300 so that the scaffold 40 thereof is temporarily exposed to the air, and causing the scaffolds 40 of the other cell culture devices 100, 200 to be constantly immersed in the culture medium 50.

Specifically, the controller 70 temporarily stops the pumps 65, 66, 67 while driving the pump 68. Then, when the liquid level of the culture medium 50 of the cell culture device 300 reaches a height position which is below the shelf 20, the controller 70 stops all the pumps 65 to 68 for a predetermined period of time (for example, 1 minute) so that only the scaffold 40 of the cell culture device 300 is exposed to the air. At this time, in the cell culture devices 100, 200, the scaffolds 40 are immersed in the culture medium 50, respectively. After the predetermined period of time passes, the controller 70 executes the control of, firstly, driving the pumps 65, 66, 67 to cause the cell culture device 300 to be filled with the culture medium 50, and then driving the pump 68 to cause the culture medium 50 to circulate through each of the cell culture devices 100, 200, 300. As described above, the fourth operation mode is an operation mode for causing only the scaffold 40 in the cell culture device 300 to be temporarily exposed to the air while and the scaffolds 40 of the other cell culture devices 100, 200 to be constantly immersed in the culture medium 50.

In the present embodiment, the cell culture system S1 includes the operation mode (first operation mode) of sequentially lowering the liquid levels of the culture medium 50 in the cell culture devices 100, 200, 300 to cause the scaffolds 40 of the cell culture devices 100, 200, 300 to be sequentially exposed to the air, and the operation modes (second to fourth operation modes) of causing only the scaffold 40 of the specific cell culture device to be exposed to the air. Switching the operation mode appropriately depending on the culture conditions enables efficient animal cell culture.

The period of time for exposing the scaffold 40 to the air can be arbitrarily set by an input operation to the controller 70 made by an operator. Specifically, in the present embodiment, the operator is allowed to change the period of time in the range from 30 seconds to 180 seconds depending on the culture conditions. Accordingly, the controller 70 appropriately controls the pumps 65 to 68 so that the scaffolds 40 of the cell culture devices 100, 200, 300 are exposed to the air for the period of time as set. It is needless to say that the flow rate of the pumps 65 to 68 can be arbitrarily set as well by an input operation to the controller 70.

### (Experiment)

The inventors conducted some experiments to investigate the extent to which the efficiency in culturing animal cells differs between a conventional operation mode and the first operation mode according to the present embodiment.

Firstly, a conventional operation mode (hereinafter, referred to as a conventional mode) will be described. In the conventional mode, the pumps 65 to 68 are driven simultaneously at a predetermined flow rate. For example, upon setting of the flow rate of the pumps 65 to 68 to 150ml/min, the controller 70 controls each of the pumps 65 to 68 to a predetermined rotational speed. This causes the culture medium 50 to circulate at 150ml/min through each of the cell culture devices 100, 200, 300 and the buffer tank 400. In this case, the liquid levels in the cell culture devices 100, 200, 300 are kept substantially constant above the shelf 20. That is, in the conventional mode, the animal cells are constantly immersed in the culture medium 50.

On the other hand, as described above, in the first operation mode of the present embodiment, the states of the animal cells (scaffold 40) in the cell culture devices 100, 200, 300 are sequentially switched between being immersed in the culture medium 50 (first state) and being exposed to the air (second state). The present inventors conducted experiments to find the extent to which the culture efficiency differs between these operation modes by measuring how much protein was to be produced in both the operation modes and compare them with each other.

The experimental conditions are as follows.
Temperature of culture medium 50: 37 degrees Celsius
pH value of culture medium 50: 7.2
Volume of culture medium 50: 350ml in each device
Mass of scaffold: 5.5g

### Experimental method:

In the conventional mode, the mass of protein contained in the culture medium 50 of the buffer tank 400 was measured in the operation performed with the scaffold 40 being constantly immersed in the culture medium 50, while the culture medium 50 was changed periodically. On the other hand, in the first operation mode, the mass of protein contained in the culture medium 50 of the buffer tank 400 was measured in the operation cycle repeatedly performed with the scaffold 40 being exposed to the air for 1 minute and then immersed in the culture medium 50 for 15 minutes, while the culture medium 50 was changed periodically.

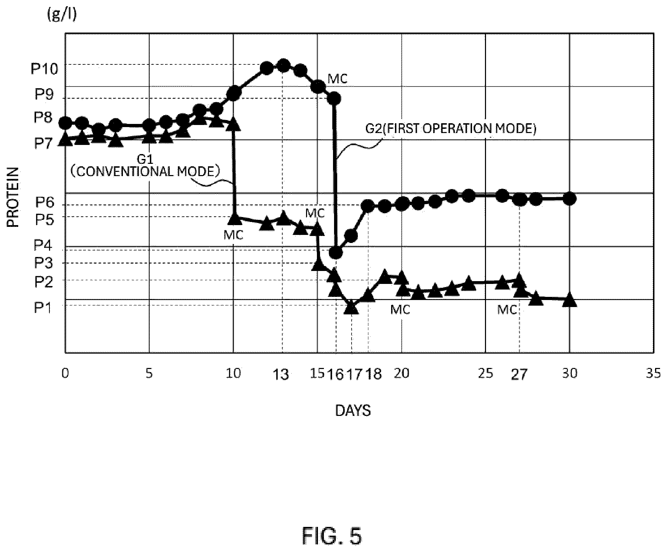

FIG. 5 illustrates the experimental results on the cell culture described above, in which the amount of protein generated in the conventional mode is compared with that in the first operation mode according to the present embodiment. In FIG. 5, the graph G1 expresses the experimental results on the conventional mode, and the graph G2 expresses the experimental results on the first operation mode. The horizontal axis of FIG. 5 represents the number of days, and the vertical axis thereof represents the mass per unit volume (g/l) of protein contained in the culture medium 50. In FIG. 5, "MC" indicates the timing at which the culture medium 50 was changed.

As illustrated in FIG. 5, in the graph G1 (conventional mode), from day 0 to day 10, the mass of protein gradually increases from P7 to P8. The culture medium 50 was changed on day 10 as the glucose contained therein was consumed, and this causes the mass of protein to rapidly decrease approximately from P8 to P5. Until day 15, the mass of protein gradually decreases, and when the culture medium 50 was changed on day 15 as the glucose contained therein was consumed, it rapidly decreases to P3. On day 17, the mass of protein decreases to P1 which is the minimum value, however, it gradually increases and reaches P2 on day 20. After the culture medium 50 was changed on day 20 and day 27 as the glucose contained therein was consumed, the mass of protein is substantially leveled in the range between P1 to P2.

On the other hand, in the graph G2 (first operation mode), from day 0 to day 10, the mass of protein gradually increases from P8 to P9, and on day 13, it reaches P10 which is the maxim value. Then, from day 13 to day 16, the mass of protein decreases. On day 16, the culture medium 50 was changed as the glucose contained therein was consumed, and this causes the mass of protein to rapidly decrease from P9 to P4. Thereafter, from day 16 to day 18, the mass of protein increases, and on day 18, it reaches P6. After day 18, the mass of protein is generally maintained at P6 without requiring change of the medium 50.

The following facts can be derived from the results of comparison between the graph G1 and the graph G2.
(1) From day 0 to day 10, there is little difference in the mass of protein contained in the culture medium 50 between the conventional mode and the first operation mode.
(2) On day 10, in the conventional mode, the culture medium 50 has to be changed as the glucose was consumed, however, in the first operation mode, the culture medium 50 did not have to be changed on day 10 as the glucose was less consumed. Furthermore, in first operation mode, the mass of protein increases even after day 10.
(3) In the conventional mode, the second change of the culture medium 50 was performed on day 15, and in the first operation mode, the first change of the culture medium 50 was performed on day 16. In both the cases, the change of the culture medium 50 causes the protein to rapidly decrease, and on day 16, almost no difference is found in the mass of protein therebetween (P2 in the conventional mode, and P4 in the first operation mode). However, after day 16, the difference in the mass of protein between the conventional mode and the first operation mode increases, and in the conventional mode, the culture medium 50 had to be changed on day 20 and day 27. On the other hand, in the first operation mode, the culture medium 50 was changed only on day 16 for the first time, but did not have to be changed thereafter.

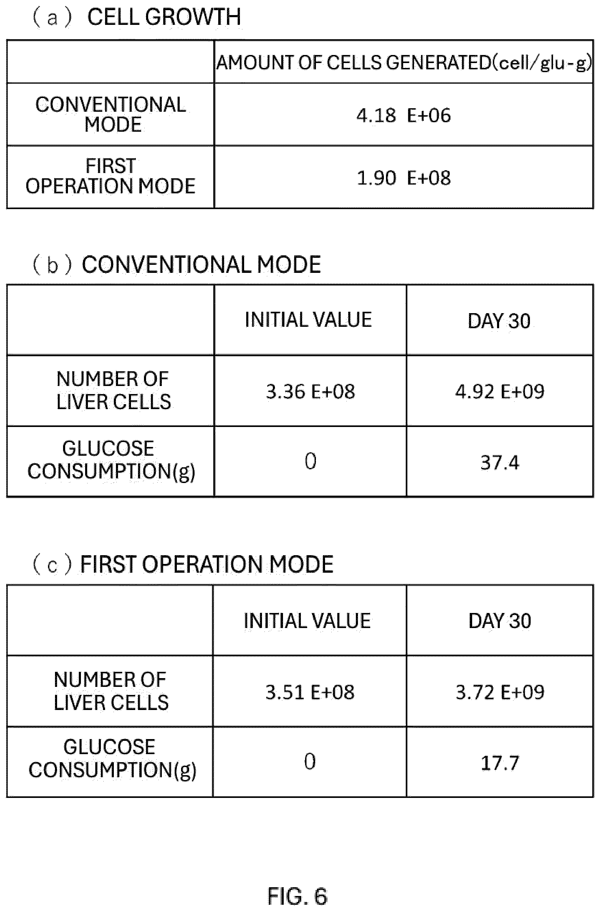

Next, the results of measurement of the consumption of glucose contained in the culture medium 50 will be described. In the experiment described above, the consumption of glucose contained in the culture medium 50 was measured in each of the conventional mode and the first operation mode. FIG. 6 illustrates the results of measurement of the number of cells in the cell culture device 200 and glucose consumption in the buffer tank 400 in the experiment described above. FIG. 6(a) illustrates the result of measurement of the amount of cells generated (the number of grown cells) in the cell culture device 200 per consumption of glucose, that is, the yield of cell growth, in each of the conventional mode and the first operation mode. FIG. 6(b) illustrates the result of measurement of the number of liver cells and glucose consumption in the conventional mode. FIG. 6(c) illustrates the result of measurement of the number of liver cells and glucose consumption in the first operation mode.

As illustrated in FIG. 6(a), in the conventional mode, the yield of cell growth is 4.18E+06 (cell/glu-g) whereas, in the first operation mode, the yield of cell growth is 1.90E+08 (cell/glu-g), from which is found that the growth in the first operation mode is approximately 45 times as much as that in the conventional mode. Furthermore, as illustrated in FIG. 6(b), in the conventional method, 37.4g of glucose is consumed to grow the liver cells from 3.36E+08 to 4.92E+09. On the other hand, as illustrated in FIG. 6(c), in first operation mode, 17.7g of glucose is consumed to grow the number of liver cells from 3.51E+08 to 3.72E+09.

Based on FIG. 6(b) and FIG. 6(c), there is no significant difference in the number of cells derived from the liver in the cell culture device 200 on day 30 between the conventional mode and the first operation mode, however, it is found that the glucose consumption (17.7g) in the first operation mode was half of the glucose consumption (37.4g) in the conventional mode. This reveals that the yield of animal cell culture in the first operation mode was clearly enhanced more than that in the conventional mode. That is, it is found that the animal cell culture efficiency is enhanced in the case of exposing the animal cells (scaffold 40) to the air for about 1 minute every 15 minutes, more than that in the case of constantly immersing the animal cells in the culture medium 50. It is considered that exposing the animal cells to the air caused the change in the metabolic states thereof, and as a result, animal cell culture efficiency increased.

Although not illustrated, the inventors performed a further experiment in the same manner as above, modifying the time for exposing the scaffold 40 (animal cells) to the air from 30 seconds to 180 seconds, from which was confirmed that the difference in the mass of protein between the conventional mode and the first operation mode shows a similar trend as that found in the experiment described above.

According to the present embodiment described above, the advantageous operations and effects as follow can be obtained.

By exposing animal cells to the air (second state) for a predetermined period of time (30 seconds to 180 seconds), the culture efficiency can be significantly improved (approximately twice). Furthermore, by connecting the cell culture devices 100, 200, 300 containing different animal cells, respectively, in a loop and circulating the culture medium 50 through each of the cell culture devices 100, 200, 300, the culture supernatant with high quality can be stored in the buffer tank 400. Still further, by controlling the liquid levels of the culture medium 50 so as to cause the animal cells of the cell culture devices 100, 200, 300 to be sequentially exposed to the air, the cells for culture meat to be cultured in each of the cell culture devices 100, 200, 300 can be efficiently obtained.

Still further, the cell culture devices 100, 200, 300 according to the present embodiment are simply designed to include only the culture tank 1 for storing the culture medium 50, the shelf 20 provided in the culture tank **1,** and the stirring means 30 for stirring the culture medium 50, and thus it can be manufactured at low cost. That is, with this simple structure, the efficiency of animal cell culture can be improved at low cost. Still further, the cell culture devices 100, 200, 300 with the simple structures can be assembled with various devices, which enables an animal cell culture system to be easily constructed.

Moreover, by simply placing the scaffold 40 on the shelf 20 and lowering the liquid level of the culture medium 50 below the shelf 20, the animal cells (scaffold 40) can be easily brought in the state (second state) where they are not immersed in the culture medium 50. Furthermore, placing the scaffold 40 on the shelf 20 allows the animal cells to easily adhere to the scaffold 40, which enhances the culture efficiency. Still further, by providing the stirring blades 33 of the stirring means 30 in a space below the shelf 20, the influence of the rotation of the stirring blades 33 on the scaffold 40 can be minimized.

In the following, this advantageous effect will be described in detail. The rotation of the stirring blades 33 causes a vortex flow at a position below the shelf 20, and the vortex flow is buffered by the nets 23 of the shelf 20 (wire mesh 22) and gently flows toward the scaffold 40. This can prevent the animal cells adhering to the scaffold 40 from detaching therefrom and thus, relax the stress on the animal cells. Moreover, the vortex flow of the culture medium 50 generated below the shelf 20 is buffered by the wire mesh 22 and then gradually rises, which enables the nutrients contained in the culture medium 50 to be uniformly supplied to the animal cells adhering to the scaffold 40.

As a result, the present embodiment can greatly shorten the time required for producing cultured meat, and therefore, contribute to the achievement of the sustainable development goals (SDGs).

Even the structure in which the stirring blades 33 are positioned above the shelf 20 can sufficiently promote the animal cell culture, however, due to a vortex flow generated above the scaffold 40, the effect of attaching the animal cells to the scaffold 40 is lower than the structure in which the stirring blades 33 are positioned below the shelf 20. Thus, the stirring blades 33 are preferably positioned below the shelf 20. Alternatively, the stirring means 30 may include the two stirring blades 33 with upper and lower blades, in which one of the stirring blades 33 is placed in a space above the shelf 20 and the other one of the stirring blades 33 is placed in a space below the shelf 20.

Furthermore, in the present embodiment, by providing the viewing window 7 through which an operator can easily observe the state in the culture tank 1, the usability can be improved.

### (Second embodiment)

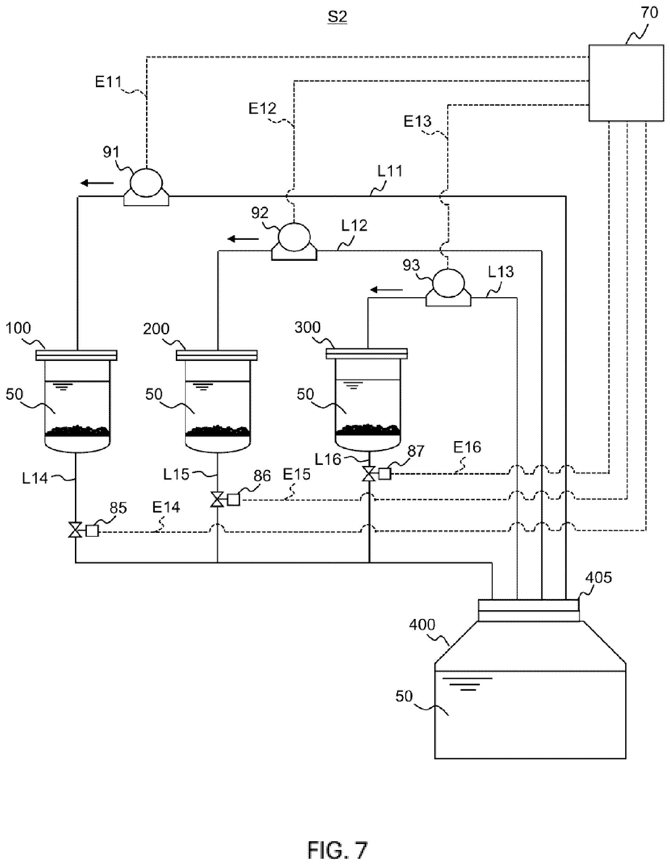

Next, a cell culture system S2 according to the second embodiment will be described. FIG. 7 illustrates an overall configuration of the cell culture system according to the second embodiment. As illustrated in FIG. 7, the features of the cell culture system S2 according to the second embodiment can be found in the arrangement in which the cell culture devices 100, 200, 300 are connected to the buffer tank 400 in parallel, respectively, so as to allow the culture medium 50 to move between the cell culture device 100 and the buffer tank 400, between the cell culture device 200 and the buffer tank 400, and between the cell culture device 300 and the buffer tank 400.

The cell culture system S2 includes a pump 91 provided in a flow path L11 between the cell culture device 100 and the buffer tank 400, a pump 92 provided in a flow path L12 between the cell culture device 200 and the buffer tank 400, and a pump 93 provided in a flow path L13 between the cell culture device 300 and the buffer tank 400. The controller 70 controls the driving of the pumps 91, 92, 93, so that the culture medium 50 in the buffer tank 400 flows through the flow paths L11, L12, L13, respectively.

The cell culture devices 100, 200, 300 and the buffer tank 400 are connected via flow paths L14, L15, L16 which are drain flow paths, and the flow paths L14, L15, L16 are provided with solenoid valves 85, 86, 87, respectively. More specifically, each of the flow paths L14, L15, L16 is formed with a pipe or hose that connects between the waste liquid nozzle 8 (see FIG. 2) provided at the bottom portion 4 of the culture tank 1 and the buffer tank 400, and these flow paths L14, L15, L16 are provided with the solenoid valves 85, 86, 87, respectively. The solenoid valves 85, 86, 87 closes in a normal state, and opens in response to a command from the controller 70 so that the culture medium 50 stored in the cell culture devices 100, 200, 300 is discharged and returns to the buffer tank 400.

Next, fifth to seventh operation modes provided in the cell culture system S2 will be described.

### (Fifth operation mode)

The fifth operation mode is a mode for lowering only the liquid level of the culture medium 50 in the cell culture device 100 so that the scaffold 40 thereof is temporarily exposed to the air, and causing the scaffolds 40 of the other cell culture devices 200, 300 to be constantly immersed in the culture medium 50.

In the fifth operation mode, the controller 70 temporarily stops the pump 91 once every 15 minutes and controls the solenoid valve 85 to fully open. This causes the culture medium 50 of the cell culture device 100 to be discharged to the buffer tank 400. Upon detecting that the culture medium 50 has reached a predetermined position which is lower than the height of the shelf 20 by means of a level sensor (not illustrated) or the like, the controller 70 controls the solenoid valve 85 to close. The controller 70 maintains the state above for, for example, 1 minute. This causes the scaffold 40 to be exposed to the air for 1 minute. This maintaining time can be flexibly changed by being set on the controller 70 by an operator.

On the other hand, the controller 70 controls the opening degree of the solenoid valves 86, 87 so as to balance with the flow rates of the pumps 92, 93, while driving the pumps 92, 93. Thus, in each of the cell culture devices 200, 300, the liquid level of the culture medium 50 is maintained at a predetermined position which is below the shelf 20, and this causes the scaffolds 40 of the cell culture devices 200, 300 to be constantly immersed in the culture medium 50.

### (Sixth operation mode)

The sixth operation mode is a mode for lowering only the liquid level of the culture medium 50 in the cell culture device 200 so that the scaffold 40 thereof is temporarily exposed to the air, and causing the scaffolds 40 of the other cell culture devices 100, 300 to be constantly immersed in the culture medium 50.

In the sixth operation mode, the controller 70 controls the pump 92 to pause once every 15 minutes and also controls the solenoid valve 86 to fully open. Thereafter, when the culture medium 50 of the cell culture device 200 reaches a predetermined position which is lower than the height of the shelf 20, the controller 70 controls the solenoid valve 86 to fully close, and maintains the closed state above, for example, for 1 minute.

On the other hand, the controller 70 controls the opening degree of the solenoid valves 85, 87, while driving the pumps 91, 93, so that the scaffolds 40 of the cell culture device 100, 300 are constantly immersed in the culture medium 50.

### (Seventh operation mode)

The seventh operation mode is a mode for lowering only the liquid level of the culture medium 50 in the cell culture device 300 so that the scaffold 40 thereof is temporarily exposed to the air, and causing the scaffolds 40 of the other cell culture devices 100, 200 to be constantly immersed in the culture medium 50.

In the seventh operation mode, the controller 70 controls the pump 93 to pause once every 15 minutes and controls the solenoid valve 87 to fully open. Thereafter, when the culture medium 50 of the cell culture device 300 reaches a predetermined position which is lower than the height of the shelf 20, the controller 70 controls the solenoid valve 87 to fully close, and maintains the closed state above, for example, for 1 minute.

On the other hand, the controller 70 controls the opening degree of the solenoid valves 85, 86, while driving the pumps 91, 92, so that the scaffolds 40 of the cell culture device 100, 300 are constantly immersed in the culture medium 50.

According to the cell culture system S2, the arrangement in which, the cell culture devices 100, 200, 300 are connected to the buffer tank 400 in parallel, respectively, allows only a particular one of the cell culture devices 100, 200, 300 to be brought in the state of being exposed to the air in accordance with the selection arbitrarily made from among the fifth to seventh operation modes. That is, exposing only the animal cells of a particular one of the cell culture devices 100, 200, 300 to the air can improve the culture efficiency.

If controlling the operation of the cell culture system S2 such that that the controller 70 repeats the fifth to seventh operation modes in order, the cell culture devices 100, 200, 300 are brought to be in the state of being exposed to the air sequentially, in the same manner as the first embodiment.

Furthermore, in the arrangement in which the cell culture devices 100, 200, 300 are connected in parallel, in addition to the fifth to seventh operation modes, a further operation mode for bringing any two of the cell culture devices 100, 200, 300 to be in the state of being simultaneously exposed to the air can be provided. Thus, using culture conditions and culture methods which are different from those in the first embodiment, the animal cell culture also can be promoted.

As described above, according to the second embodiment, in the same manner as the first embodiment, it is possible to efficiently culture different types of animal cells and to obtain the culture supernatant rich in active ingredients.

The present invention is not limited to the embodiments described above, and various modifications are possible within the scope of not deviating from the concept of the present invention. All of the technical matters included in the technical concept described in the scope of the claims are the subject of the present invention. The embodiments described above exemplify the preferred examples, and those skilled in the art can make various alternatives, modifications, variations, and improvements based on the disclosure herein, all of which would fall within the scope of the claims as attached.

For example, the present invention is also applicable as a device for culturing cells other than cells derived from an animal (for example, cells derived from a plant). In the case of using the small-sized culture tank 1, for example, a magnetic stirrer may be used as the stirring means 30. Using a magnetic stirrer, the structure can be advantageously simplified.

The shelf 20 may include, instead of the wire mesh 22, a punching plate having a plurality of holes. In this case, the size of the hole may be, for example, about 100 microns in diameter. The hole may be formed in any shape, for example, it may be a round hole, a square hole, or a long hole. Furthermore, as the shelf 20, a plate having a large number of slits may be used. Still further, the plurality of shelves 20 may be provided in the culture tank 1 in multiple stages. In this case, the size of the nets 23 (that is, mesh size) or the hole diameter may differ on each of the shelves 20.

The number of cell culture devices is not limited to three, and the number of buffer tanks 400 is not limited to one. The buffer tank 400 may not be incorporated into the system. Furthermore, in each of the embodiments, all the operation modes described above do not necessarily have to be provided. That is, depending on the culture conditions, the cell culture system may be provided with a part of the operation modes.

Still further, the same animal cells may be supplied to the cell culture devices 100, 200, 300, respectively, or different animal cells may be supplied to only a part of the cell culture devices 100, 200, 300.

### REFERENCE SIGNS LIST

1 culture tank
2 flange
3 body portion
4 bottom portion
5 lid body
5a-5h mounting holes
6 leg
7 viewing window
8 waste liquid nozzle
9 flange
10 blind flange
12 protrusion
16 culture medium supply pipe
17 culture medium transfer pipe
20 shelf
21 ring
22 wire mesh
23 net
24a-24d small hole
25 central hole
27 leg portion
30 stirring means
31 motor
32 rotation shaft
33 stirring blade
40 scaffold
50 culture medium
65-68 pump
70 controller
80-82 balance
85-87 solenoid valve
91-93 pump
100, 200, 300 cell culture device
S1, S2 cell culture system

## Claims

1. A cell culture system comprising a plurality of cell culture devices which are connected such that a culture medium moves through each of the plurality of cell culture devices, wherein
the liquid level of the culture medium stored in each of the plurality of cell culture devices is controlled such that cells in at least one of the plurality of cell culture devices are in a first state, while cells in the remaining cell culture devices are in a second state, the first state being a state in which the cells are immersed in the culture medium, and the second state being a state in which the cells are not immersed in the culture medium.

2. The cell culture system according to claim 1, wherein
the plurality of cell culture devices is connected in a loop, and the culture medium circulates through each of the plurality of cell culture devices.

3. The cell culture system according to claim 2, further comprising a buffer tank for storing the culture medium therein, wherein the buffer tank is connected among the plurality of cell culture devices.

4. The cell culture system according to any one of claims 1 to 3, wherein
the at least one of the plurality of cell culture devices includes:
a culture tank for storing the culture medium therein;
a shelf for partitioning the culture tank in a height direction, the shelf having an opening through which the culture medium passes; and
a stirring means for stirring the culture medium, and
the shelf allows a scaffold to which the cell is attached to be placed thereon.

5. The cell culture system according to claim 4, wherein
when the at least one of the plurality of cell culture devices is in the second state, the liquid level of the culture medium is maintained at a position below the shelf.

6. The cell culture system according to claim 5, wherein
the period of time during which the second state is maintained ranges from 30 seconds to 180 seconds.

7. Cultured meat produced by the cell culture system according to any one of claims 1 to 3.

8. Culture supernatant produced by the cell culture system according to any one of claims 1 to 3.
